(19) Europäisches Patentamt
European Patent Office
Office européen des brevets

(11) Veröffentlichungsnummer: **0 116 967**
**B1**

# (12) EUROPÄISCHE PATENTSCHRIFT

(45) Veröffentlichungstag der Patentschrift: **29.01.86**

(51) Int. Cl.⁴: **C 07 C 103/82**, A 61 K 31/165

(21) Anmeldenummer: **84101647.0**

(22) Anmeldetag: **17.02.84**

(54) **N-Phenyl-benzamid-Derivate, Verfahren zu deren Herstellung und diese enthaltende Arzneimittel.**

(30) Priorität: **19.02.83 DE 3305755**

(43) Veröffentlichungstag der Anmeldung: **29.08.84 Patentblatt 84/35**

(45) Bekanntmachung des Hinweises auf die Patenterteilung: **29.01.86 Patentblatt 86/5**

(84) Benannte Vertragsstaaten: **AT BE CH DE FR GB IT LI LU NL SE**

(56) Entgegenhaltungen:
**GB - A - 1 123 457**
**GB - A - 1 509 035**
**US - A - 3 488 388**

**CHEMICAL ABSTRACTS, Band 96, Nr. 26, 28. Juni 1982, Seite 377, Nr. 223265y, Columbus, Ohio, USA**

(73) Patentinhaber: **GÖDECKE AKTIENGESELLSCHAFT, Salzufer 16, D-1000 Berlin 10 (DE)**

(72) Erfinder: **Satzinger, Gerhard, Dr., Im Mattenbühl 7, D-7809 Denzlingen (DE)**
Erfinder: **Herrmann, Manfred, Dr., Wolfweg 25, D-7811 St. Peter (DE)**
Erfinder: **Fritschi, Edgar, Dr., Am Scheuerwald 2, D-7811 St. Peter (DE)**
Erfinder: **Weiershausen, Ute, Bundes-Strasse 70, D-7803 Gundelfingen (DE)**

**EP 0 116 967 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Aus der US-PS 39 26 922 sind Diamino-benzanilid-Derivate als Ausgangsprodukte für die Herstellung von Polyurethan-Elastomeren bekannt geworden. Eine pharmakologische Wirkung dieser Verbindungsklasse ist jedoch bisher nicht beschrieben worden.

Aus der US-PS 3 488 388 sind ebenfalls Diamino-benzanilid-Derivate bekannt geworden. Diese sind aber im Benzoylrest ortho-substituiert und nur als Antioxidantien oder Mittel gegen Ozon brauchbar.

Es wurde gefunden, dass Verbindungen der allgemeinen Formel I

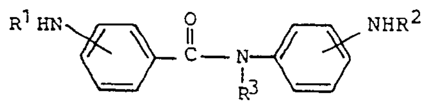 (I)

in welcher die Reste $R^1$, $R^2$ und $R^3$, die gleich oder verchieden sein können, ein Wasserstoffatom oder eine Methylgruppe bedeuten können, sowie deren Salze mit pharmakologisch unbedenklichen Säuren interessante pharmakologische Eigenschaften besitzen, welche sie besonders zur Behandlung maligner, proliferativer und autoimmuner Erkrankungen sowie zur immunosuppressiven Therapie bei Tansplantationen geeignet machen.

Bei denjenigen Verbindungen der allgemeinen Formel I, welche als Reste $R^1$ und/oder $R^2$ wenigstens eine Methylgruppe besitzen, handelt es sich um *neue* Verbindungen, welche durch die folgende allgemeine Formel II charakterisiert sind:

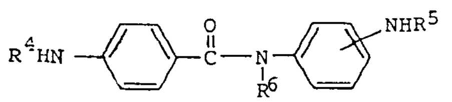 (II)

in welcher mindestens einer der Reste $R^4$ und $R^5$ eine Methylgruppe darstellt, und die übrigen Reste $R^4$ bis $R^6$, die gleich oder verschieden sein können, ein Wasserstoffatom oder eine Methylgruppe bedeuten.

Gegenstand der vorliegenden Erfindung sind somit neue Verbindungen der allgemeinen Formel II sowie Arzneimittel mit einem Gehalt an Verbindungen der allgemeinen Formel I zur Behandlung von Erkrankungen des Immunsystems und zur immunosuppressiven Therapie.

Die Verbindungen der allgemeinen Formel II können hergestellt werden, indem man Verbindungen der allgemeinen Formel III a und b:

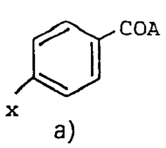 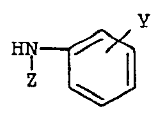 (III a) + b))

a) b)

in welcher A eine reaktive Säuregruppe, Z ein Wasserstoffatom oder eine Methylgruppe und X und Y, die gleich oder verschieden sein können, eine mit einer Schutzgruppe versehene Aminogruppe oder eine Nitrogruppe bedeuten, in an sich bekannter Weise miteinander umsetzt, die so erhaltenen Verbindungen der allgemeinen Formel IV,

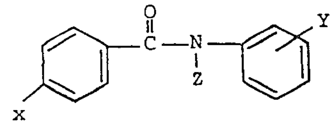 (IV)

in welcher X, Y und Z die obengenannte Bedeutung haben, mit Wasserstoff umsetzt und gewünschtenfalls hydrolysiert und anschliessend mit Säuren in deren pharmakolisch verträgliche Salze überführt.

Besonders bevorzugt sind Verbindungen der allgemeinen Formeln I und II, in welchen die Reste -NH-$R^1$ bzw. -NH-$R^4$ sich in para-Stellung zur Carbonylgruppe befinden.

Als Einzelsubstanzen werden aufgrund der guten pharmakologischen Wirkung folgende besonders bevorzugt:

– 4-Amino-N-(2'-aminophenyl)-benzamid
– 4-Methylamino-N-(2'-aminophenyl)-benzamid
– 4-Amino-N-(2'-methylaminophenyl)-benzamid
– 4-Amino-N-(2'-aminophenyl)-N-methylbenzamid

Als Salze kommen alle therapeutisch verwendbaren Säureadditionssalze in Betracht, wie z.B. Salze mit Halogenwasserstoffsäuren, wie Chlor- oder Bromwasserstoffsäure, Schwefelsäuren, Phosphorsäuren, Salpetersäure, aliphatischen, alicyclischen, aromatischen oder heterocyclischen Carbon- oder Sulfonsäuren, wie Ameisen-, Essig-, Propion-, Bernstein-, Glykol-, Milch-, Äpfel-, Wein-, Zitronen-, Ascorbin-, Malein-, Hydroxymalein- oder Brenztraubensäure; Phenylessig-, Benzoe-, p-Aminobenzoe-, Anthranil-, p-Hydroxybenzoe-, Salicyl- oder p-Aminosalicylsäure, Methansulfon-, Äthansulfon-, Hydroxyäthansulfon-, Äthylensulfonsäure; Halogenbenzolsulfon-, Toluolsulfon-, Naphthalinsulfonsäure oder Sulfanilsäure; Methionin, Tryptophan, Lysin oder Arginin.

Die erfindungsgemässen Wirkstoffe werden vorteilhaft in Form eines pharmazeutischen Präparats verabreicht, welches die Wirkstoffe in freier Form oder in Form einer ihrer therapeutisch verwendbaren Salze, in Mischung mit einem z.B. für die topische, enterale, z.B. orale oder rectale, oder vor allem parenterale, wie intramuskuläre oder intravenöse Applikation geeigneten pharmazeutischen organischen oder anorganischen, festen oder flüssigen Trägermaterial enthält. Für die Bildung derselben kommen solche Stoffe in Frage, die mit den neuen Verbindungen nicht reagieren, wie z.B. Gelatine, Lactose, Stärke, Stearylalkohol, Magnesiumstearat, Talk, pflanzliche Öle, Benzylalkohole, Propylenglykole, Vaseline oder andere Arzneimittelträger.

Die pharmazeutischen Präparate können z.B. als Tabletten, Dragées, Kapseln, Suppositorien, Salben, Cremes oder in flüssiger Form als Lösungen, Suspensionen oder Emulsionen vorliegen. Gegebenenfalls sind sie sterilisiert und/oder enthalten Hilfsstoffe, wie Konservierungs-, Stabilisierungs-, Netz- oder Emulgiermittel, Lösungsvermittler oder Salze zur Veränderung des osmotischen Druckes

oder Puffer. Sie können auch weitere Wirkstoffe enthalten.

Die anzuwendende Dosis hängt von der Art des zu therapierenden Krankheitsgeschehens und von individuellen Faktoren ab.

Im allgemeinen werden Dosen von 10 bis 300 mg, insbesondere 20 bis 100 mg verabreicht. In besonderen Fällen kann die Einzeldosis auch höher liegen.

Die Umsetzung der Verbindungen gemäss Formel IIIa mit Verbindungen der Formel IIIb erfolgt in an sich bekannter Weise. Als reaktive Säuregruppen A dienen vor allem die Säurehalogenide, -anhydride oder -imidazolide oder Estergruppen, die eine Umsetzung mit der Aminogruppe erlauben. A bedeutet somit bevorzugt Halogen, Imidazolyl-, Acyl- oder niedere Alkylreste.

Als Schutzgruppen für X und/oder Y dienen die auch in der Peptidchemie üblichen Reste, wie z.B. der Acetyl-, der Benzyl- oder der Carbobenzoxy-Rest.

Die Umsetzung mit Wasserstoff wird unter Verwendung geeigneter Katalysatoren (Platin oder Palladium) so durchgeführt, dass einerseits die freien Nitrogruppen zu primären Aminogruppen reduziert werden und andererseits die an den Aminogruppen befindlichen Schutzgruppen hydrogenolytisch abgespalten werden. Im Falle der Anwesenheit einer Acetylgruppe wird diese hydrolytisch, z.B. mit Salzsäure abgespalten. Auf diese Weise gelangt man je nach Wunsch zu methylsubstituierten oder unsubstituierten Aminogruppen $R^1$-NH- oder $R^4$-NH-.

Die erfindungsgemässen Substanzen besitzen überraschende pharmakologische Wirkungen und sind insbesondere Mittel zur Immunsuppression/Immunregulation bei Haut-, Organ- und Knochenmarkstransplantationen, zur Immunsuppression/Immunregulation bei Störungen des zellulären und humoralen Immunsystems (z.B. Autoaggressionserkrankungen, Immunkomplexerkrankungen, Erkrankungen des rheumatischen Formenkreises usw.), zur topischen und/oder systemischen Behandlung maligner Neoplasien (einschliesslich solcher des hämatopoetischen Systems, der Haut, des ZNS) und zur topischen und/oder systemischen Behandlung benigner proliferativer Erkrankungen (z.B. Psoriasis) dar. Aufgrund der nachgewiesenen cytostatischen Wirkung wird auch eine antivirale Wirkung in Betracht gezogen.

Die Verbindungen der allgemeinen Formel I unterscheiden sich von bekannten Cytostatika und Immunsuppressiva insbesondere durch ihre Lipophilie, die in der Eigenart ihrer chemischen Struktur begründet ist, durch ihre Potenz, ihre topische Aktivität und durch die Steuerbarkeit von Wirkung und Nebenwirkung aufgrund einer besonderen Wirkungskinetik.

Im Unterschied zu den meisten Substanzen, die aufgrund ihrer hydrophilen Eigenschaften die Bluthirnschranke nicht passieren und daher eine befriedigende therapeutische Beeinflussung z.B. von malignen Tumoren des Zentralnervensystems nicht erlauben, gelangen die erfindungsgemässen Substanzen infolge ihres günstigen Verteilungskoeffizienten (Log $V_K$ von Beispiel 1 = 2.2, n-Octanol/Wasser pH 7,4/20° C) in das ZNS. Dies ist auch bei intragastraler Applikation der Fall, wie bestimmte pharmakologische Effekte zeigen.

Durch das Passieren der Bluthirnschranke eröffnen sich somit bisher nicht gegebene Möglichkeiten einer therapeutischen Beeinflussung proliferativer wie auch autoimmuner Prozesse im ZNS. Weiterhin besteht bei einem lipophilen Cytostatikum die Möglichkeit der Anreicherung in besonders fettreichen Tumoren, wodurch eine effektivere medikamentöse Beeinflussung solcher Neoplasien erzielt werden kann.

Vergleichende Untersuchungen der erfindungsgemässen Substanzen gegen den immunsuppresiven Standard Azathioprin zeigen ihre Überlegenheit auf: So sind zur Erzielung einer etwa gleichen Wirkung auf die Überlebenszeit allogener Hauttransplantate der Ratte im Falle von Azathioprin im Vergleich zu den Verbindungen der Formel I um 300 bis 700% höhere Dosen erforderlich.

Über eine topische antiproliferative Aktivität, wie sie für die erfindungsgemässen Substanzen gezeigt werden kann, wurde bei herkömmlichen Cytostatika bisher nicht berichtet.

Es wurde weiterhin gefunden, dass die suppressive Wirkung auf periphere Leuko- und Lymphozyten des Rhesusaffen bei Absetzen einer der erfindungsgemässen Substanzen rasch reversibel ist. Eine derart kurzfristige weitgehende Wiederherstellung normaler hämatologischer Befunde ist bei den herkömmlichen Cytostatika und Immunsuppressiva (z.B. Cyclophosphamid, Methotrexat, Azathioprin) nicht zu beobachten. Hieraus kann geschlossen werden, dass Überdosierungen bedingt durch individuell unterschiedliches Ansprechen und daraus resultierende Nebenwirkungen besser zu kontrollieren sind als bei herkömmlichen Cytostatika und Immunsuppressiva.

In der Immunsuppression werden Steroide, Azathioprin, Antilymphozyten-Globulin (ALG), Anti-Thymozyten-Globulin (ATG) und Cyclosporin A eingesetzt. Die Nebenwirkungen dieser Therapien sind beträchtlich, die Komplikationen gefährlich. Die Verbindungen der vorliegenden Anmeldung sind extrem nebenwirkungsarm und bedeuten allein deswegen einen grossen therapeutischen Fortschritt.

Die derzeitige Psoriasis-Medikation muss als ausserordentlich unbefriedigend bezeichnet werden. Zu den wirksamsten Antipsoriatika gehören Corticoide. Systemisch müssen sie jedoch so hoch dosiert werden, dass Steroid-Toxizität häufig ist. Nach dem Absetzen kann es zu schweren Rückfällen oder gar Umwandlungen in die chronischen pustulösen Formen kommen. Rückfälle treten auch nach topischer Anwendung von Corticoiden auf.

Der Folsäureantagonist Methotrexat gilt ebenfalls als ein wirksames Antipsoriatikum. Methotrexat gilt als wirksames Mittel bei mässiger bis schwerer Psoriasis-Arthritis. Wegen des hohen Risikos der Hepato-, Myelo- und gastrointestinalen Toxizität wird die Methotrexat-Behandlung jedoch auf akut an generalisierten schweren Formen der Psoriasis erkrankte Patienten beschränkt. Die Verbindungen der vorliegenden Anmeldung wirken proliferationshemmend auch bei topischer Applikation, so dass sie ausser für die systemische Behandlung der Psoriasisarthritis auch für die topische Behandlung der psoriatischen Hauterscheinungen Bedeutung erlangen können. Nebenwirkungen, wie sie die im Handel befindlichen Antipsoriatika aufweisen, werden aufgrund der chemischen Eigenschaften der erfindungsgemässen Substanzen nicht erwartet.

Der Entwicklung lipophiler Cytostatika muss hohe Bedeutung beigemessen werden, da die bekannten Substanzen nicht oder in therapeutisch nicht ausreichenden Konzentrationen in das ZNS gelangen und daher eine zufriedenstellende cytostatische Medikation maligner Tumoren des ZNS zur Zeit nicht möglich ist. Als besonders interessante Indikation für ein lipophiles Cytostatikum kommen dabei insbesondere die Leukämien in Frage, da diese häufig ins ZNS disseminieren, von wo aus der Krankheitsprozess weiter unterhalten wird. Ein weiterer möglicher Vorteil eines Cytostatikums mit lipophilen Eigenschaften besteht in der Anreicherung in besonders fettreichen Tumoren, die daher evtl. bevorzugt beeinflusst werden können.

Die folgenden Vergleichsversuche belegen die Wirkung der Verbindungen der allgemeinen Formel I:

*Vergleichsversuche*

I. *Untersuchung der immunsuppressiven Aktivität im Hauttransplantat-Überlebenstest bei Ratten*

Die erfindungsgemässe Substanz A (4-Amino-N-(2'-aminophenyl)-benzamid) wurde auf immunsuppressive Aktivität im Hauttransplantationsmodell bei Ratten und Rhesusaffen geprüft; sie wurde in den Experimenten an Ratten gegen den internationalen immunsuppressiven Standard *Azathioprin* bzw. gegen eine derzeit bei Organtransplantationen allgemein gebräuchliche Kombinationsmedikation, bestehend aus hochdosiertem *Azathioprin und niedrigdosiertem Methylprednisolon,* verglichen. Substanz A war in diesen Versuchen mindestens gleich wirksam wie die Vergleichsmedikation, obwohl weit geringere Substanzmengen eingesetzt wurden. Auch am Rhesusaffen war die immunsuppressive Wirkung deutlich ausgeprägt. Wie an dieser Spezies gezeigt, ist die mit Substanz A bewirkte Verlängerung der normalen Überlebenszeit allogener Hauttransplantate durch gleichzeitig ausgeprägte Senkung der peripheren Lymphozyten gekennzeichnet.

A) *Technik*

*Vergleichsmedikation*: Versuch I/Azathioprin + Methylprednisolon
Versuch II/Azathioprin

*Zubereitung*: Als Suspension jeweils frisch vor der Verabreichung

*Applikationsart*: intragastral per Schlundsonde, suspendiert in 0,8% Methocel

*Volumen*: 0,01 ml je Gramm Körpergewicht und Dosis

*Dosierungsschema*: Versuch I/ab Tag −3 vor Transplantation bis Tag + 6
Versuch II/ab Tag −2 vor Transplantation bis Tag + 9

*Geprüfte Dosen*: Versuch I/10 mg/kg über 6 Tage, danach 5 mg/kg tägl. über 4 Tage
Versuch II/4-8 mg/kg tägl. steigend über 12 Tage

B) *Modellbeschreibung*

Es werden jeweils 2 allogene Hauttransplantate einer Spenderratte auf eine Empfängerratte eines nicht verwandten Inzuchtstammes übertragen. Dabei kann der dem Transplantat anhaftende Panniculus carnosus präparativ entfernt (suprapanniculäres Transplantat) oder am Transplantat belassen werden (subpanniculäres Transplantat). Die Transplantation erfolgt am Tag 0 in leichter Äthernarkose nach vorangegangenem Scheren und Reinigen des Operationsfeldes. Die Transplantate werden im Transplantatbett durch Anlegen einer geeigneten Bandage fixiert. Ab Tag +9 wird den Tieren ein Schutzkragen angelegt, die Bandage abgenommen und der Zustand der Transplantate täglich kontrolliert. Die Beurteilung der Transplantatfunktion erfolgt anhand einer Graduierungsskala, die eine Differenzierung entsprechend 3 Schweregraden der Abstossung erlaubt. Endpunkt ist der Tag, an dem 50% der Tiere einer Gruppe beide Transplantate abgestossen haben. Es wird die Verlängerung des Transplantatüberlebens im Vergleich zu den Kontrollen in Tagen bestimmt.

C) *Ergebnisse*

Wie in den nachfolgenden Tabellen I und II zu entnehmen ist, war in Versuch I die Substanz A einer immunsuppressiven Standardmedikation, bestehend aus Azathioprin und Methylprednisolon, überlegen. Im Versuch II war die Substanz A gleich wirksam wie der immunsuppressive Standard Azathioprin. Aufgrund der hohen Aktivität der Substanz A waren jedoch zur Erzielung der Wirkung im Vergleich zur Standardmedikation weit geringere Substanzmengen erforderlich.

*Tabelle I* zeigt die Ergebnisse des Versuches I. Hauttransplantat-Überlebenstest, Ratte.

Empfänger: Long Evans ♂, Spender: Lewis ♂.

Es wurde eine Übertragung von 2 suprapanniculären Transplantaten eines Spenders auf einen Empfänger vorgenommen.

*Tabelle II* zeigt die Ergebnisse des Versuches II. Hauttransplantat-Überlebenstest, Ratte.

Empfänger: Osborne Mendel ♂, Spender: Lewis ♂.

Es wurde die Übertragung von 2 subpanniculären Transplantaten eines Spenders auf einen Empfänger durchgeführt.

*Tabelle I*

| Prüfsubstanz, Dosierung | Tiere mit 2 abgestossenen Transplantaten/n (%) | | | | | | | | |
|---|---|---|---|---|---|---|---|---|---|
| | Tag +9 | Tag +10 | Tag +11 | Tag +12 | Tag +13 | Tag +14 | Tag +15 | Tag +16 | Tag +17 |
| *Kontrolle* (Methocel i.g.) | 1/6 (17) | 3/6 (50) ** | 4/6 (66) | 4/6 (66) | 4/6 (66) | 5/6 (83) | 6/6 (100) | — | — |
| *Substanz A*** 10 mg/kg i.g. ab Tag −3 bis Tag +2, danach 5 mg/kg i.g. ab Tag +3 bis Tag +6 | 0/5 ( 0) | 0/5 ( 0) | 0/5 ( 0) | 0/5 ( 0) | 1/5 (20) | 1/5 (20) | 3/5 ( 60) * | 4/5 (80) | 5/5 (100) |
| *Azathioprin* 40 mg/kg i.g.+Methylprednisolon 2 mg/kg i.g. ab Tag −3 bis Tag +6 | 0/6 ( 0) | 0/6 ( 0) | 1/6 (17) | 2/6 (33) | 2/6 (33) | 4/6 (66) * | 6/6 (100) | — | — |

* Tag der Abstossung: mindestens 50% der Tiere haben beide Transplantate abgestossen
** Substanz A: 4-Amino-N-(2′-aminophenyl)-benzamid (Beispiel 1)

*Tabelle II*

| Prüfsubstanz, Dosierung | Tiere mit 2 abgestossenen Transplantaten/n (%) | | | | |
|---|---|---|---|---|---|
| | Tag +8 | Tag +9 | Tag +10 | Tag +11 | Tag +12 |
| *Kontrolle* (Methocel i.g.) | 2/10 (20) | 5/10 (50) * | 6/10 (60) | 8/10 (80) | 9/10 (90) |
| *Substanz A*** 4-8 mg/kg i.g. ab Tag −2 bis Tag +9 | 0/10 ( 0) | 1/10 (10) | 1/10 (10) | 1/10 (10) | 6/10 (60) * |
| *Azathioprin* 20-60 mg/kg i.g. ab Tag −2 bis Tag +9 | 0/10 ( 0) | 1/10 (10) | 2/10 (20) | 4/10 (40) | 5/10 (50) * |

* Tag der Abstossung: mindestens 50% der Tiere haben beide Transplantate abgestossen
** Substanz A: 4-Amino-N-(2′-aminophenyl)-benzamid (Beispiel 1)

II. *Untersuchung von Substanz A auf immunsuppressive Aktivität im Hauttransplantat-Überlebenstest bei Rhesusaffen (Macaca mulatta)*

Der Nachweis der Wirkung im Hauttransplantationsmodell ist als Nachweis einer Inhibition des zellulären Immunsystems (T-Lymphozyten) zu werten, da dieses nach derzeit gültiger Lehrmeinung an der Rejektion allogener Transplantate massgeblich beteiligt ist.

Auf eine Inhibition des humoralen Immunsystems (Antikörper produzierende B-Lymphozyten) durch die erfindungsgemässen Substanzen und eine sich daraus ergebende Therapiemöglichkeit z.B. von Autoaggressionskrankheiten, die durch eine überschiessende Antikörperproduktion gegen körpereigenes Gewebe gekennzeichnet sind, kann derzeit nur indirekt geschlossen werden. Als indirekter Nachweis für die Inhibition der humoralen Immunabwehr ist eine bei längerfristig

mit hohen Dosen der Substanz A behandelten Ratten beobachtete erhöhte Anfälligkeit gegenüber bakteriellen Infekten anzusehen.

A) *Technik*

*Vergleichsstandard*: Aus ethischem Grund nicht eingesetzt
*Zubereitung*: Jeweils frisch vor der Verabreichung
*Applikationsart*: intragastral per Schlundsonde in 0,8% Methocel suspendiert
*Dosierungsschema*: 1 × täglich über 10 Tage
*Geprüfte Dosis*: 10 mg/kg

B) *Versuchsbeschreibung*

Es werden insgesamt 4 allogene Hauttransplantate von 2 verschiedenen Spendertieren kreuzweise auf 1 nicht verwandtes Empfängertier übertragen. Zunächst werden die Transplantate in Ketamin-Narkose von der geschorenen, gereinigten Bauchhaut der Spendertiere entnommen und von anhaftendem Fett befreit. Die vier Transplantatbetten werden auf dem geschorenen, gereinigten Rücken des Empfängertieres in Ketamin-Narkose durch Entnahme von Hautstücken geeigneter Grösse vorbereitet. Nach erfolgter Blutstillung werden die Transplantate im Transplantatbett ausgebreitet und mit dessen Wundrändern vernäht. Die Wunde wird mit geignetem Wundmaterial abgedeckt und dies mit einer elastischen Binde fixiert. An den Tagen 5, 7, 9 nach der Transplantation wird die Bandage geöffnet und die Funktion der Transplantate überprüft. Am 9. Tag wird die Bandage nicht mehr angelegt. Es wird dann eine tägliche Kontrolle der Transplantatfunktion bis zur erfolgten Abstossung vorgenommen.

C) *Ergebnisse*

In diesem Versuch an Primaten werden Kontrollen aus ethischem Grund nicht mitgeführt. Es wird gegen das normale Überleben allogener Hauttransplantate historischer Kontrollen verglichen, das mit 10 Tagen angegeben wird. Mit Substanz A konnte in der geprüften Dosierung eine Verlängerung des normalen Transplantatüberlebens bei Versuchstier ZE um 6-7 Tage, bei Versuchstier YL um 4-5 Tage erzielt werden. Dieses Ergebnis ist als klare immunsuppressive Wirkung der erfindungsgemässen Substanz zu interpretieren.

Die Figuren 1 und 2 zeigen den Einfluss einer 10tägigen Medikation von Substanz A auf Leukozyten und Lymphozyten des Plasmas bei Rhesusaffen.

Dosierung: 1 × täglich 10 mg/kg i.g.
Fig. 1: Rhesusaffe ZE, ♀
Fig. 2: Rhesusaffe YL, ♀

III. *Untersuchung der antineoplastischen Aktivität von Substanz A in 3 Tumormodellen bei Mäusen*

Bei intragastraler Verabreichung von Substanz A an tumortragende Mäuse zeigten diese im Vergleich zu nicht behandelten Kontrolltieren in Abhängigkeit von dem verwendeten Tumormodell eine signifikante Zunahme der mittleren Überlebenszeit bzw. eine deutliche Wachstumsverzögerung des betreffenden Tumors.

Topische Auftragungen von Substanz A in geeignetem Lösungsmittel führten bei Ratten zu einer deutlichen Inhibition des wachsenden Haarfollikels.

Die Inhibition des wachsenden Haarfollikels ist eine oft beobachtete und in der Literatur beschriebene Nebenwirkung der systemischen Cytostatika-Medikation. Sie ist Ausdruck der antiproliferativen Wirkung dieser Substanzklasse, von der die rasch proliferierenden Gewebe besonders betroffen sind.

A) *Technik*

*Zubereitung*: Als Suspension jeweils frisch vor der Verabreichung
*Applikationsart*: oral, suspendiert in 0,8% Methocel
*Volumen*: 0,01 ml je Gramm Körpergewicht und Dosis
*Dosierungsschema*: täglich über 9 Tage, sofern nicht anders angegeben
*Geprüfte Dosis*: 60, 120, 180, 240 mg/kg täglich

B) *Tumorbeschreibung*

*L 1210/Rij* Adaption von L 1210 Leukämie bei $CD_2$ Mäusen (C57B1 × DBA2, F1 Hybride). Am Tag vor der Substanzgabe werden $10^5$ leukämische Zellen intraperitoneal inokuliert. Die Käfige werden 2 × täglich auf verendete Tiere kontrolliert. Endpunkt ist das mediane Überleben von 5 Mäusen/Dosis im Vergleich zu 10 unbehandelten Kontrollen.

*Osteosarkom* C22LR bei BCBA Mäusen (C57BL × CBA/Rij, F1 Hybride). Der Tumor wird subcutan bilateral in die Flanken inokuliert. Er wird mindestens 2 × wöchentlich in 3 Dimensionen gemessen. Endpunkt ist die Wachstumsverzögerung. Sie ist definiert als die Differenz der durchschnittlichen Zeit, in welcher das Volumen von 5 bis 10 Tumoren bei 5 behandelten Tieren im Vergleich zu dem von 5-10 Tumoren bei den Kontrolltieren einen bestimmten Wert erreicht. Dieser Wert wurde festgelegt als das 4fache des mittleren Volumens bei Behandlungsbeginn.

*Lewis Lung Tumor* bei BCBA Mäusen. Das Vorgehen gleicht dem bei Osteosarkom. Hier wurde das Tumorvolumen mit 800 $mm^3$ (= Produkt der Durchmesser in 3 Dimensionen) festgelegt.

C) *Ergebnisse*

Aufgrund der Toxizität der ausgewählten Dosen waren tägliche Applikationen in den meisten Dosisgruppen nicht möglich. Nachdem Mortalität in allen Gruppen nach 2 oder mehr Applikationen von 120 mg/kg oder mehr aufgetreten war, wurde die Anzahl der Verabreichungen bei den überlebenden Tieren reduziert. Die Befunde sind den nachfolgenden Tabellen III bis V zu entnehmen.

Beurteilung: Die geprüften Dosierungen wurden zu hoch angesetzt. Trotzdem konnte eine signifikante Aktivität der geprüften Substanz bei

L 1210 in mässiger Dosierung, eine gute Aktivität bei Osteosarkom und eine mässige Aktivität bei Lewis Lung Tumor nachgewiesen werden.

Die nachfolgenden Tabellen III bis V geben die Ergebnisse der Testung von Substanz A auf neoplastische Aktivität bei der Maus wieder:

*Tabelle III*

| L 1210 | | |
|---|---|---|
| Dosierung | Mediane Überlebenszeit (Tage) | % Zunahme der medianen Überlebenszeit |
| Kontrollen (n=10) | 8 | — |
| *Testgruppen:* | | |
| 9× 60 mg/kg p.o. (n= 5) | 11 | 38% |
| 7×120 mg/kg p.o. (n= 5) | 6 | toxischer Tod |
| 4×180 mg/kg p.o. (n= 5) | 5 | toxischer Tod |
| 4×240 mg/kg p.o. (n= 5) | 5 | toxischer Tod |

*Tabelle IV*

| Osteosarkom | | |
|---|---|---|
| Dosierung | Überlebende Tiere | Wachstumsverzögerung des Tumors (Tage) |
| Kontrollen (n=5) | 5/5 | — |
| *Testgruppen:* | | |
| 9× 60 mg/kg i.p. (n=5) | 5/5 | 7,8 (p=0,0001) |
| 4×120 mg/kg p.o. (n=5) | 0/5 (Tag 5) | toxischer Tod |
| 2×180 mg/kg p.o. (n=5) | 1/5 | 9,6 |
| 1×240 mg/kg p.o. (n=5) | 5/5 | 3,1 (p=0,001) |

*Tabelle V*

| Lewis Lung Tumor | | |
|---|---|---|
| Dosierung | Überlebende Tiere | Wachstumsverzögerung des Tumors (Tage) |
| Kontrollen (n=5) | 5/5 | — |
| *Testgruppen:* | | |
| 9× 60 mg/kg i.p. (n=5) | 5/5 | 3,8 (p=0,061) |
| 4×120 mg/kg p.o. (n=5) | 0/5 | toxischer Tod |
| 2×180 mg/kg p.o. (n=5) | 0/5 | toxischer Tod |
| 1×240 mg/kg p.o. (n=5) | 5/5 | 3,5 (p=0,018) |

IV. *Untersuchung von Substanz A auf antiproliferative Aktivität bei topischer Verabreichung an Ratten*

A) *Technik*

*Vergleichsstandard*: Kein Vergleichsstandard bekannt

*Zubereitung*: Jeweils frisch vor der Verabreichung

*Applikationsart*: Topisch durch Auftropfen mittels Pipette als 0,5%ige Lösung in Ethanol absolut

*Volumen*: 1 ml je Tier und Dosis

*Geprüfte Dosen*: 5 mg/Tier täglich über 14 Tage, danach 10 mg/Tier täglich über 7 Tage

B) *Versuchsbeschreibung*

Männlichen Ratten des Stammes Long Evans, Fellfarbe «Schwarzhooded», wird ein Schutzkragen angelegt. Auf der Rückenhaut wird in der Region des schwarzen Rückenhaarstreifens nach dem Scheren und Reinigen mit Hilfe einer Schablone ein Rechteck von 3 cm Länge und 1,5 cm Breite markiert. Das Auftragen der Testlösung auf dieses Rechteck wird langsam und tropfenweise vorgenommen, um ein Abfliessen auf die umgebende Haut zu vermeiden. Bei den Kontrolltieren wird das Lösungsmittel in gleicher Weise appliziert. Nach 21 Behandlungstagen wird der Haarwuchs auf den behandelten Feldern im Vergleich zu den Kontrollfeldern beurteilt.

C) *Ergebnisse*

An den beobachteten Tieren lässt sich eine deutliche Inhibition des Haarwuchses bei den behandelten Tieren im Vergleich zu den Kontrolltieren erkennen.

Beurteilung: Obwohl nicht auszuschliessen ist, dass ein Teil der verabreichten Substanz infolge einer möglichen perkutanen Resorption auf systemischem Wege an der Inhibition des wachsenden Haarfollikels beteiligt war, konnte gezeigt werden, dass mit der erfindungsgemässen Substanz eine deutliche antiproliferative Wirkung auf Anhanggebilde der Haut bei topischer Applikation erzielt wird.

V) *Zentrale Wirkungen von Substanz A*

Die Penetrationsfähigkeit der erfindungsgemässen Substanzen in das ZNS und andere lipoide Organe lässt sich auch durch ihre ausgeprägten antikonvulsiven Effekte im maximalen Elektrokrampf-Test (Swinyard et al., J. Pharm. exp. Ther. *106*, 319 (1952) und im Pentetrazolkrampf-Modell (loc. cit.) aufzeigen:

*Antikonvulsive Wirkung von Substanz A an Mäusen*

*Tabelle VI*

| Krampfmodell | Substanz A<br>ED 50 mg/kg (i.g.) | Diphenylhydantoin<br>ED 50 mg/kg (i.g.) |
|---|---|---|
| Elektroschock | 30,0 (21-42) | 22,5 (18-28) |
| Pentetrazolkrampf | 44,0 (32-60) | 9,5 ( 7-13) |

Um die Grössenordnung der zentralen Effekte anschaulich zu machen, wurde das Standard-Antiepilepticum Diphenylhydantoin als Referenzsubstanz herangezogen.

Die 7-Tage-Werte der akuten, oralen (i.g.) Toxizität von Substanz A und von Diphenylhydantoin an der Maus sind in der Tabelle VII vergleichsweise aufgeführt:

*Tabelle VII*

| | LD 50 (mg/kg) | Vertrauensgrenze in mg/kg p=0,05 | |
|---|---|---|---|
| | | untere | obere |
| Substanz A | 625 | 469,9 | 831,2 |
| Diphenylhydantoin | 128 | 114,2 | 143,3 |

Das folgende Beispiel dient der näheren Erläuterung der Erfindung:

*Beispiel 1*

*4-Amino-N-(2'-aminophenyl)-benzamid*

$C_{13}H_{13}N_3O$ MG 227.26

22,7 g o-Nitroanilin, gelöst in 130 ml wasserfreiem Dioxan, werden tropfenweise mit einer Lösung von 30,4 g p-Nitrobenzoylchlorid in 150 ml trockenem Dioxan versetzt. Die Reaktion wird durch 1-stündiges Erhitzen unter Rückfluss zu Ende geführt. Beim Abkühlen auf 15° C fällt N-(2'-Nitrophenyl)-4-nitrobenzamid in kristalliner Form aus. Nach dem Umkristallisieren aus Chlorbenzol erhält man 39 g (83% d.Th.) von Schmp. 217-218° C.

39 g des Dinitroproduktes werden in 550 ml Dimethylformamid in Gegenwart von 10 g Raney-Ni 1 Stunde bei 25° C und 2 Atü hydriert. Man filtriert vom Katalysator ab, entfernt das Solvens i. Vak. und kristallisiert das Titelprodukt aus Ethanol um. Ausbeute 20,2 g (66% d.Th.) vom Schmp. 180-181° C.

Die Verbindung lässt sich mittels einer Lösung von HCl-Gas in Ethanol in das Di-Hydrochlorid überführen, welches zu 4% wasserlöslich ist.

*4-Amino-N-(2'-aminophenyl)-benzamid-dihydrochlorid*

$C_{13}H_{15}N_3Cl_2O$ MG 300.18
Schmp. 340° C (aus Ethanol)

*Beispiel 2*

*4-Methylamino-N-(2'-aminophenyl)-benzamid*

2,71 g N-(2-Nitrophenyl)-4-methylamino-benzamid werden in 300 ml Tetrahydrofuran gelöst und in einer Schüttelapparatur mit Raney-Nickel hydriert. Während der Hydrierung steigt die Temperatur von 21° C auf 26° C. Nach dem Abfiltrieren vom Katalysator wird die klare Lösung eingedampft und der Rückstand aus Essigester umkristallisiert.

Man erhält 1,3 g (56,5% d.Th.) 4-Methylamino-N-(2'-aminophenyl)-benzamid; Fp. 189° C.

Das als Ausgangsprodukt eingesetzte N-(2-Nitrophenyl)-4-methylamino-benzamid wird wie folgt hergestellt:

4,535 g (0,03 Mol) 4-Methylamino-benzoesäure werden unter Rühren mit 9,19 g (0,09 Mol)

Acetanhydrid 1 Stunde auf 100° C erhitzt. Die nach dem Erkalten der klaren Lösung ausgefallenen Kristalle werden abgesaugt und mit Essigester nachgewaschen (Fp. 199-201° C). Die erhaltene N-Acetyl-4-methylamino-benzoesäure (Ausbeute 62%) wird dann mittels Thionylchlorid in Dioxan in das Säurechlorid übergeführt. Überschüssiges Thionylchlorid wird dann mit etwas Dioxan im Wasserstrahlvakuum abgezogen. Zum Reaktionsgemisch werden 2,76 g (0,02 Mol) o-Nitroanilin in 10 ml trockenem Dioxan getropft und die Lösung mit 4,04 g (0,04 Mol) Triethylamin (als HCl-Fänger) versetzt. Dann wird 1 Stunde am Rückfluss gekocht. Der eingedampfte Reaktionsrückstand wird mit 40 ml Wasser versetzt und mit Methylenchlorid extrahiert. Nach Einengen der organischen Phase werden 4 ml Isopropanol zugegeben und die gebildeten Kristalle nach 30 minütigem Rühren abgesaugt.

Man erhält 5,1 g N-(2-Nitrophenyl)-4-(N-acetyl-N-methylamino)-benzamid (Fp. 162° C) in einer Ausbeute von 55,6% d.Th. Das so erhaltene Rohprodukt wird dann in wenig Ethanol suspendiert und mit der siebenfachen molaren Menge konz. Salzsäure 9 Stunden am Rückfluss hydrolysiert. Die Reaktionsmischung wird zum Trocknen eingeengt und durch Zugabe von wenig Isopropanol zur Kristallisation gebracht, in 40 ml Wasser gelöst und mit Ammoniak basisch gestellt. Nach Extraktion mit Methylenchlorid erhält man 1,4 g N-(2 - Nitrophenyl) - 4 - methylamino-benzamid, Fp. 187° C in einer Ausbeute von 51% d.Th.

In analoger Weise erhält man die folgenden Verbindungen:

*4-Amino-N-(2'-methylaminophenyl)-benzamid*

*4-Methylamino-N-(2'-methylaminophenyl)-benzamid*

*Beispiel 3*

*4-Amino-N-(2'-aminophenyl)-N-methylbenzamid*

22,82 g (0,15 Mol) N-Methyl-o-nitroanilin werden in 70 ml trockenem Tetrahydrofuran gelöst und unter Rühren und Durchblasen von Stickstoff tropfenweise mit 30,6 g (0,165 Mol) p-Nitrobenzoylchlorid in 90 ml trockenem Tetrahydrofuran versetzt. Es wird dann unter Stickstoff 2 Stunden am Rückfluss weiter gerührt. Das erhaltene Reaktionsgemisch wird eingeengt und aus Essigester umkristallisiert.

Man erhält 29,2 g (64,6% d.Th.) N-Methyl--N-(2-nitrophenyl)-4-nitrobenzamid, Fp. 137-138° C, welches in 800 ml THF gelöst und mit 10 g Raney-Nickel in der Schüttelapparatur unter Wasserkühlung hydriert wird. Hierbei soll die Temperatur nicht über 25° C steigen. Nach 7 Stunden werden nochmals 10 g Raney-Nickel zugegeben und nach weiteren 6 Stunden wird die farblose Lösung zur Trockene eingeengt und der Rückstand in Essigester gelöst und mit Diisopropyläther gefällt.

Man erhält 18 g 4-Amino-N-(2'-aminophenyl)-N-methyl-benzamid; Fp. 159-160° C.

In analoger Weise erhält man folgende Verbindungen:

*4-Methylamino-N-(2'-aminophenyl)-N-methylbenzamid*

*4-Methylamino-N-(2'-methylaminophenyl)-N-methylbenzamid*

*4-Amino-N-(2'-methylaminophenyl)-N-methylbenzamid*

**Patentansprüche** für die Vertragsstaaten BE, CH, DE, FR, GB, IT, LI, LU, NL, SE

1.) Verbindungen der allgemeinen Formel II

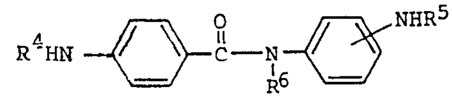

(II)

in welcher mindestens einer der Reste $R^4$ und $R^5$ eine Methylgruppe darstellt und die anderen Reste $R^4$ bis $R^6$, die gleich oder verschieden sein können, ein Wasserstoffatom oder eine Methylgruppe bedeuten,
sowie deren pharmakologisch verträglichen Salze mit anorganischen oder organischen Säuren.

2.) Arzneimittel enthaltend mindestens eine Verbindung der allgemeinen Formel I

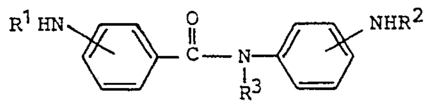

(I)

in welcher die Reste $R^1$, $R^2$ und $R^3$, die gleich oder verschieden sein können, ein Wasserstoffatom oder eine Methylgruppe bedeuten,
oder deren pharmakologisch verträgliche Salze zur Bekämpfung von malignen, proliferativen und autoimmunen Erkrankungen sowie zur immunosuppressiven Therapie bei Transplantationen.

3.) Arzneimittel gemäss Anspruch 2, enthaltend 4-Amino-N-(2'-aminophenyl)-N-benzamid oder dessen pharmakologisch verträgliche Salze.

4.) Verfahren zur Herstellung von Verbindungen der allgemeinen Formel II, dadurch gekennzeichnet, dass man Verbindungen der allgemeinen Formel III a und b

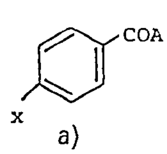

a)

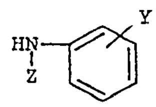

b)

(III a) + b))

in welcher A eine reaktive Säuregruppe, Z ein Wasserstoffatom oder eine Methylgruppe und X und Y, die gleich oder verschieden sein können, eine mit einer Schutzgruppe versehene Aminogruppe oder eine Nitrogruppe bedeuten,
in an sich bekannter Weise miteinander umsetzt, die so erhaltenen Verbindungen der allgemeinen Formel IV,

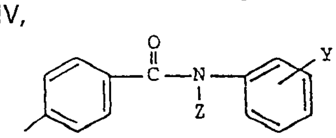

(IV)

in welcher X, Y und Z die obengenannte Bedeutung haben, mit Wasserstoff umsetzt und gewünschtenfalls hydrolysiert und anschliessend

mit Säuren in deren pharmakologisch verträgliche Salze überführt.

**Patenanspruch** für den Vertragsstaat AT

Verfahren zur Herstellung von Verbindungen der allgemeinen Formel II,

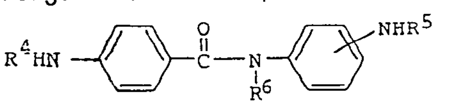
(II)

in welcher mindestens einer der Reste $R^4$ und $R^5$ eine Methylgruppe darstellt und die anderen Reste $R^4$ bis $R^6$, die gleich oder verschieden sein können, ein Wasserstoffatom oder eine Methylgruppe bedeuten,
sowie deren pharmakologisch verträglichen Salze mit anorganischen oder organischen Säuren dadurch gekennzeichnet, dass man Verbindungen der allgemeinen Formel III a und b

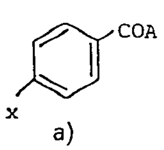
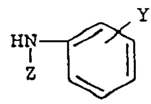
(III a) + b))

a) b)

in welcher A eine reaktive Säuregruppe, Z ein Wasserstoffatom oder eine Methylgruppe und X und Y, die gleich oder verschieden sein können, eine mit einer Schutzgruppe versehene Aminogruppe oder eine Nitrogruppe bedeuten, in an sich bekannter Weise miteinander umsetzt, die so erhaltenen Verbindungen der allgemeinen Formel IV,

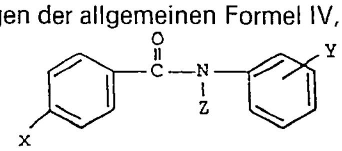
(IV)

in welcher X, Y und Z die obengenannte Bedeutung haben, mit Wasserstoff umsetzt und gewünschtenfalls hydrolisiert und anschliessend mit Säuren in deren pharmakologisch verträgliche Salze überführt.

**Claims** for the contracting States: BE, CH, DE, FR, GB, IT, LI, LU, NL, SE

1. Compounds of the general formula II

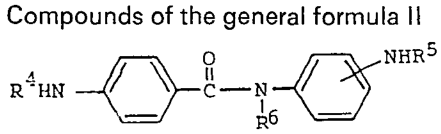
(II)

wherein at least one of the radicals $R^4$ and $R^5$ represents a methyl radical, and the other radicals $R^4$ to $R^6$, which can be the same or different, are a hydrogen atom or a methyl radical, as well as the pharmacologically acceptable salts thereof with inorganic or organic acids.

2. Pharmaceutical containing at least one compound of the general formula I

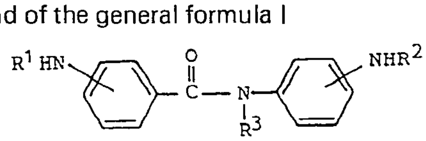
(I)

wherein the radicals $R^1$, $R^2$ and $R^3$, which can be the same or different, are a hydrogen atom or a methyl radical, as well as of the pharmacologically acceptable salts thereof for controlling malignant, proliferative and autoimmune diseases and for immunosuppressive therapy with transplantations.

3. Pharmaceutical according to Claim 2 containing 4-amino-N-(2'-aminophenyl)-N-benzamide or the pharmacologically acceptable salts thereof.

4. Process for the preparation of compounds of the general formula II, in which compounds of the general formulas III a and b

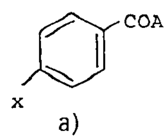
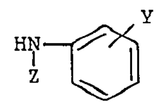
(III a) + b))

a) b)

wherein A is a reactive acid group, Z a hydrogen atom or a methyl group and X and Y, which can be the same or different, are an amino group provided with a protective group or a nitro group, are reacted with one another in a previously known manner, the compounds thus obtained of the general formula IV,

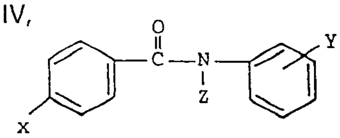
(IV)

wherein X, Y and Z have the aforementioned meanings, are reacted with hydrogen and, if desired, are hydrolysed and subsequently converted by means of acids into the pharmacologically acceptable salts thereof.

**Claim** for the contracting State: AT

Process for the preparation of compounds of the general formula II,

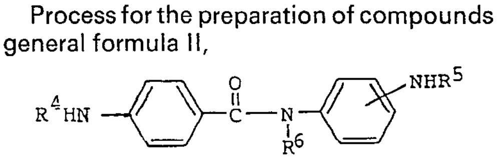
(II)

wherein at least one of the radicals $R^4$ and $R^5$ represents a methyl radical, and the other radicals $R^4$ to $R^6$, which can be the same or different, are a hydrogen atom or a methyl radical, as well as of the pharmacologically acceptable salts thereof with inorganic or organic acids, in which compounds of the general formulas III a and b

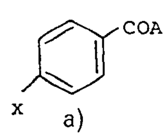
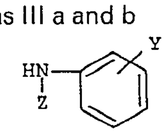
(III a) + b))

a) b)

wherein A is a reactive acid group, Z a hydrogen atom or a methyl group and X and Y, which can be the same or different, are an amino group provided with a protective group or a nitro group, are reacted with one another in a previously known manner, the compounds thus obtained of the general formula IV,

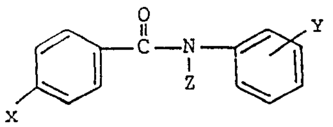

(IV)

wherein X, Y and Z have the aforementioned meanings, are reacted with hydrogen and, if desired, are hydrolysed and subsequently converted by means of acids into the pharmacologically acceptable salts thereof.

**Revendications** pour les Etats contractants: BE, CH, DE, FR, GB, IT, LI, LU, NL, SE

1. Les composés de formule générale II

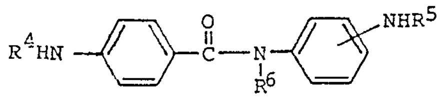

(II)

dans laquelle au moins l'un des radicaux $R^4$ et $R^5$ représente un groupe méthyle et les autres radicaux $R^4$ à $R^6$, qui peuvent être identiques ou différents, représentent un atome d'hydrogène ou un groupe méthyle, ainsi que leurs sels pharmaceutiquement acceptables d'acides minéraux ou organiques.

2. Le médicament contenant au moins un composé de formule générale I

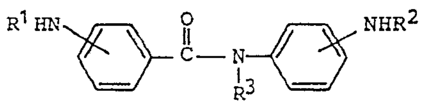

(I)

dans laquelle les radicaux $R^1$, $R^2$ et $R^3$, qui peuvent être identiques ou différents, représentent un atome d'hydrogène ou un groupe méthyle, ou leurs sels pharmaceutiquement acceptables pour la lutte contre les maladies malignes, prolifères et auto-immunes ainsi que pour la thérapie immunosuppressive lors des transplantations.

3. Le médicament selon la revendication 2, qui contient du 4-amino-N-(2'-aminophényl)-N-benzamide ou ses sels pharmaceutiquement acceptables.

4. Le procédé de préparation de composés de formule générale II, caractérisé en ce que l'on fait réagir l'un avec l'autre de façon connue en soi des composés de formule générale III a et b

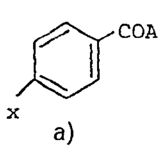

a)

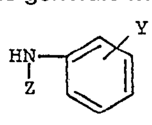

b)

(III a) + b))

dans laquelle A représente un groupe réactif acide; Z représente un atome d'hydrogène ou un groupe méthyle; et X et Y, qui peuvent être identiques ou différents, représentent un groupe amino pourvu d'un groupe protecteur, ou un groupe nitro, on fait réagir les composés ainsi obtenus de formule IV,

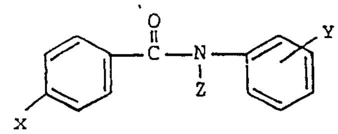

(IV)

dans laquelle X, Y et Z ont la signification indiquée ci-dessus, avec l'hydrogène et, le cas échéant, on procède à leur hydrolyse et on les transforme ensuite à l'aide d'acides en leurs sels pharmaceutiquement acceptables.

**Revendication** pour l'Etat contractant: AT

Le procédé de préparation de composés de formule générale II

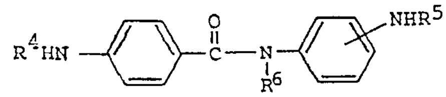

(II)

dans laquelle au moins l'un des radicaux $R^4$ et $R^5$ représente un groupe méthyle et les autres radicaux $R^4$ à $R^6$, qui peuvent être identiques ou différents, représentent un atome d'hydrogène ou un groupe méthyle, ainsi que leurs sels pharmaceutiquement acceptables d'acides organiques ou minéraux, caractérisé en ce que l'on fait réagir de façon connue en soi l'un avec l'autre des composés de formule générale III a et b

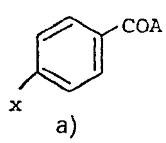

a)

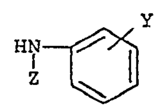

b)

(III a) + b))

dans laquelle A représente un groupe acide réactif; Z représente un atome d'hydrogène ou un groupe méthyle; et X et Y, qui peuvent être identiques ou différents, représentent un groupe amino pourvu d'un groupe protecteur, ou un groupe nitro, on fait réagir les composés ainsi obtenus de formule générale IV,

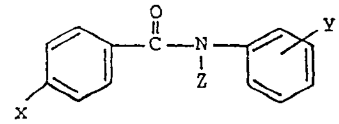

(IV)

dans laquelle X, Y et Z ont la signification indiquée ci-dessus, avec de l'hydrogène et l'on procède, le cas échéant, à leur hydrolyse et on les transforme ensuite avec des acides en leurs sels pharmaceutiquement acceptables.

# Fig. 1

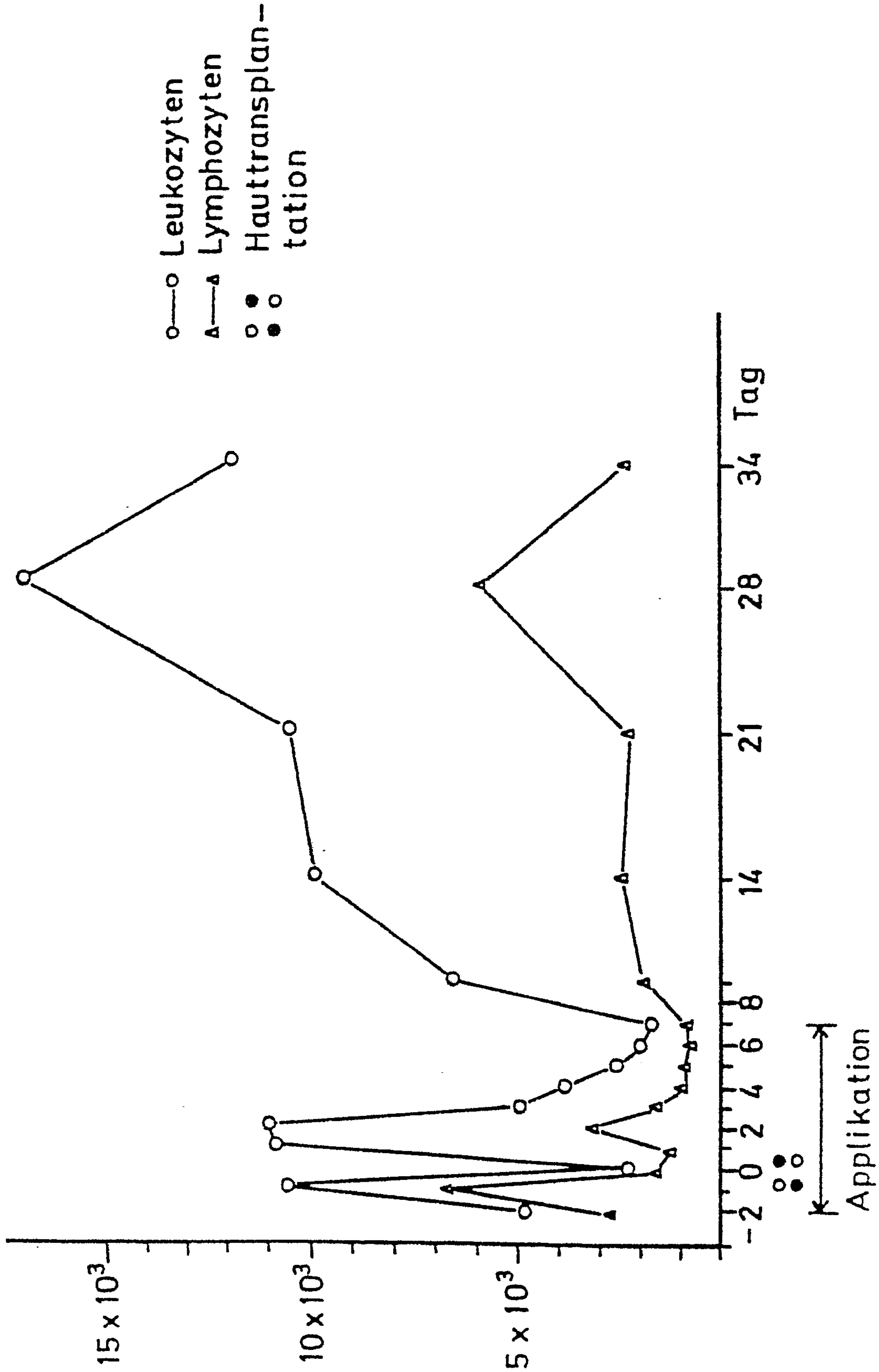

Leukozyten
Lymphozyten
Hauttransplan-
tation
15 x 10$^3$
10 x 10$^3$
5 x 10$^3$
-2
0
2
4
6
8
14
21
28
34
Tag
Applikation

Fig. 2

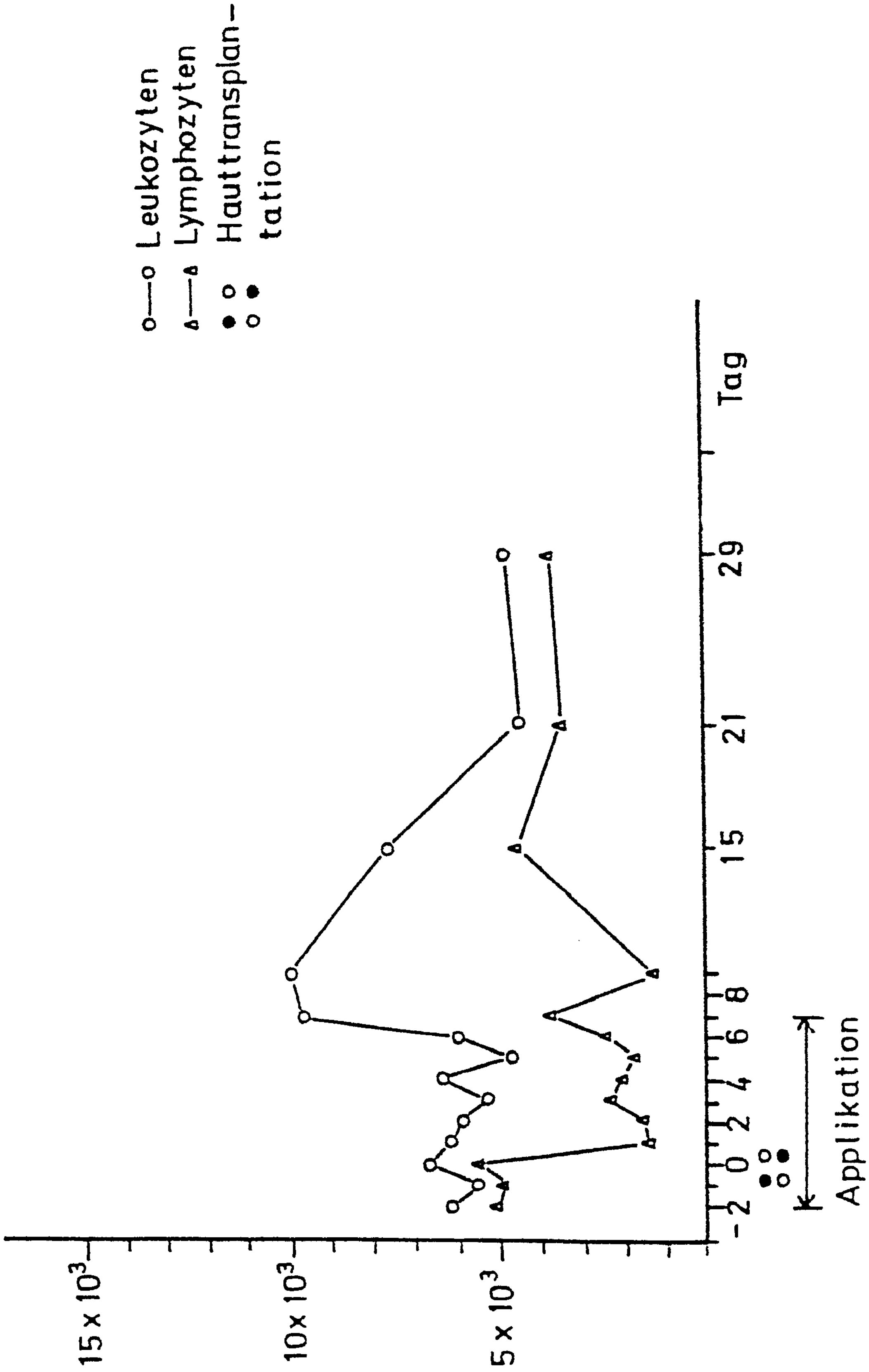
Leukozyten
Lymphozyten
Hauttransplan-
tation
Tag
-2
0
2
4
6
8
15
21
29
15 x 10³
10 x 10³
5 x 10³
Applikation